# EUROPEAN PATENT SPECIFICATION

(11) **EP 1 238 651 B1**
(45) Date of publication and mention of the grant of the patent: **05.10.2005**
(21) Application number: 02075793.6
(22) Date of filing: 27.02.2002
(51) Int. Cl.: A61K 7/42

(54) **Agents for potentiating preservatives in sunscreen formulations**
Agentien zur Potenzierung der Wirksamkeit von Konservierungsmitteln in Sonnenschutzformulierungen
Agents pour renforcer la puissance des conservateurs dans des formulations d'ecrans solaires

(30) Priority: 27.02.2001 EP 01200736
(43) Date of publication of application: 11.09.2002
(73) Proprietor: Johnson & Johnson Consumer France SAS, 92787 Issy les Moulineaux Cedex 9 (FR)
(72) Inventor: Morelli, Muriel, 27400 Acquniy (FR); Brillouet, Anne-Sophie, 27400 Louviers (FR); Porrachia, Jean-François, 78200 Mantes La Joie (FR)
(74) Representative: Weber-Bruls, Dorothée

(56) References cited:
- EP-A- 1 172 086
- WO-A-98/47469
- US-A- 5 648 083
- PATENT ABSTRACTS OF JAPAN vol. 1996, no. 06, 28 June 1996 (1996-06-28) & JP 08 053338 A (SHISEIDO CO LTD), 27 February 1996 (1996-02-27)
- DATABASE WPI Section Ch, Week 200146 Derwent Publications Ltd., London, GB; Class D21, AN 2001-427538 XP002201376 & JP 2001 048720 A (ASAHI DENKA KOGYO KK), 20 February 2001 (2001-02-20)

## Description

### Field of the invention

This invention relates to chemical compositions containing caprylyl glycol or an analog thereof, and a particular combination of preservative agents. It further relates to the use of caprylyl glycol or an analog thereof to increase the activity of preservative agents, more in particular to increase the activity of a combination of iodopropynyl butyl carbamate and phenoxyethanol.

### Background of the invention

Public awareness of the risks related to the exposure of the skin to sunlight currently is higher than ever before and in particular the harm caused by the ultraviolet spectrum of solar radiation has become well recognized. One of the more important factors that contributed thereto is the increased incidence of skin conditions such as actinic keratosis and carcinomas such as malignant melanoma in many countries over the last decades.

Obviously, there are several options to protect oneself from the harm caused by exposure, in particular by intense exposure, of the skin to sunlight. The simplest method is to avoid direct exposure of the skin to solar radiation. For many people this is not desirable or feasible for a number of reasons. Therefore a plurality of sunscreen products, also referred to as sun protection products, has been developed.

Initially these products contained chemical substances that absorb mainly UV-B rays, but with the recognition that sunlight in the UV-A region causes adverse effects on the human skin too, newer products containing filters against both UV-A and UV-B have been developed.

Sunscreen products have become widely spread in the market place and are available in a wide variety of forms such as lotions, oils, creams and the like, as well as with different sun protection factors (SPF). During the last couple of years the focus has been on the development of sunscreen products with still higher protective factors.

State of the art sunscreen products comprise either only organic filters, or inorganic pigments, or a combination of both. To achieve the currently desired high protective factors fairly high amounts of these filters have to be incorporated in the formulations.

As with most if not all personal care products, sunscreen products also contain preservatives.

A disadvantage associated with the use of preservatives in sunscreen products, and in particular with the use of phenoxyethanol and/or iodopropynyl butylcarbamate is that effectiveness of the preservatives and in particular of the latter preservatives is reduced to an unacceptable level. This in turn creates a risk of the formulation being affected by microorganisms.This is especially the case with sunscreen products that contain inorganic pigments, in particular titanium dioxide pigments.

An obvious way to tackle this problem would be by increasing the amount of preservative or preservatives. This however is not desirable since this would cause adverse effects such as allergic responses and irritation. This is especially the case when applying the products on sensitive skins or on sunburned skins.

Therefore it would be desirable to provide sunscreen products, and in particular to provide formulations or compositions for use in sunscreen products, that on the one hand are microbiologically safe and are not susceptible to microbiological contamination and deterioration and this for a sustained period of time, while on the other are devoid of adverse effects.

WO 97/20464 describes bactericidal compositions which comprise a synergistic mixture of 2-methyl-4,5-trimethylene-4-isothiazoline-3-on; 3-iodo-2-propynyl butyl carbamate and 2-phenoxyethanol. WO 98/47469 describes preservative systems comprising iodopropynyl butyl carbamate and phenoxyethanol.

JP 0805338 A is about a dermal agent of external use having improved security while keeping enough preservability, which besides moisture keeping agents, emollients, surfactants, thickeners and sequestering agents comprises hexylene glycol and phenoxyethanol as a preservative mixture. According to JP 13048720 A 1,2-octanediol can increase the humectant effect of cosmetics and can give also a bacteriostatic effect to said cosmetics when used in amounts of about 0,01 to 10 wt.% while exhibiting a lower skin irritation than that of p-hydroxybenzoic ester.

The compositions or formulations provided in the present invention meet these goals in that the effect of the preservative agents is potentiated so that less of the preservative needs to be used. Furthermore the beneficial effect of the potentiating agent extends also to the broadness of the anti-microbial spectrum.

Quite unexpectedly it has been found that the use of caprylyl glycol (and/or analogs thereof) enhances the efficacy of a preservative agent combination comprising phenoxyethanol and iodopropynyl butylcarbamate.

Caprylyl glycol is a material that is currently used as humectant or emollient in cosmetic applications or is used to combat microorganisms in cosmetics (see for example JP20011048720).

### Summary of the Invention

Thus in one aspect the present invention is concerned with chemical compositions containing caprylyl glycol or an analog thereof, and a combination of preservative agents, namely a combination of phenoxyethanol and iodopropynyl butylcarbamate.

In a further aspect, the invention concerns formulations for topical or dermatological application containing a chemical composition as defined herein. Or, alternatively the invention concerns topical formulations containing caprylyl glycol, iodopropynyl butyl carbamate and phenoxyethanol. The topical formulations of the invention can be for dermatological use, but in particular are for cosmetic use. Preferred are topical formulations which are sunscreen formulations.

In a further aspect there are provided sunscreen products comprising a sunscreen formulation as defined herein.

In a further aspect, the invention relates to the use of caprylyl glycol or an analog thereof, for potentiating or boosting the effect of a combination of the preservative agents phenoxyethanol and iodopropynyl butylcarbamate in topical formulations, in particular in sunscreen formulations but also in dermatological or in cosmetic formulations.

In still a further aspect the invention concerns the use as a preservative agent of a combination of caprylyl glycol or an analog thereof and a combination of iodopropynyl butylcarbamate and phenoxyethanol, the preservative or the latter being present in an amount that is less than effective.

The invention further provides a composition as defined herein, wherein the iodopropynyl butylcarbamate and/or phenoxyethanol are present in a concentration that is less effective.

The invention further relates to the use of a composition as defined herein as a preservative system. Said use preferably is in topical formulations, in particular in sunscreen formulations.

### Detailed Description of the Invention

As used herein all percentages are w/w.

Furthermore, as used herein, caprylyl glycol refers to 1,2-octanediol and can be structurally represented by the formula:

CH₃-(CH₂)₄-CH₂-CH(OH)-CH₂OH

Caprylyl glycol analogs comprise C₅₋₂₀ alkanediols, in particular C₆₋₁₆ alkanediols, more in particular C₆₋₁₂ alkanediols, wherein the hydroxy groups are vicinally substituted and wherein one hydroxy is substituted at an end carbon and the other on the carbon atom next thereto. Examples of such alkanediols are 1,2-nonanediol, 1,2-heptanediol and the like.

A particular group of caprylyl glycol analogs are those which have a 1,2-octanediol skeleton which is further substituted with 1, 2 or 3 C₁₋₄ alkyl groups (or 1,2-octanediol substituted with 1, 2 or 3 C₁₋₄ alkyl groups) such as, for example, 3-methyl-1,2-octanediol, 4-methyl-1,2-octanediol, 3,4-dimethyl octanediol, 3-ethyl-1,2-octanediol, 4-ethyl-1,2-octanediol and the like.

The caprylyl glycol analogs that can be used in the compositions or formulations of the invention preferably are devoid of any adverse effects on the skin, in particular on atopical skins, such adverse effects particularly comprising allergic reactions and irritation.

The term 'caprylyl glycol or an analog thereof' is also meant to comprise mixtures of caprylyl glycol and one or more of its analogs, or mixtures of two or more caprylyl glycol analogs, in particular those analogs mentioned herein. The term 'caprylyl glycol' when used in isolation is also meant to comprise caprylyl analogs, in particular the analogs mentioned herein. The term 'caprylyl glycol' when used in isolation is also meant to comprise mixtures of caprylyl glycol and one or more caprylyl glycol analogs, or mixtures of two or more caprylyl glycol analogs, in particular those analogs mentioned herein.

One preservative that is used in the products or compositions of the invention is phenoxyethanol, which can be represented by the formula:

C₆H₅O-CH₂-CH₂-OH

A second preservative iodopropynyl butylcarbamate, also referred to as butyl-3-iodo-2-propynylcarbamate or 'IPBC', and has the following chemical structure:

I-C=C-CH₂-O-(C=O)-NH-(CH₂)₃-CH₃

Preferred are mixtures of iodopropynyl butylcarbamate and phenoxyethanol wherein the weight ratio of iodopropynyl butylcarbamate to phenoxyethanol may vary but preferably is within the range of about 1:90 to about 1:400, preferably within the range of about 1:90 to about 1:200, more preferably within the range of about 1:90 to about 1:150 or to about 1:110. Particularly preferred is a weight ratio of iodopropynyl butylcarbamate to phenoxyethanol of about 1:100.

The weight by weight ratio of caprylyl glycol or its analog to the preservative agent mixture as defined herein typically is in the range of 0.1 to 500, in particular 1 to 500, or 1 to 250, or 2 to 500. Preferably said ratio is in the range of 5 to 100, or 10 to 100, or 5 to 75. More preferably said ratio is in the range of 20 to 100, or 20 to 75. Still more preferably said ratio is in the range of 25 to 75 or 50 to 60. In particular said ratio is 55 or about 55.

In the compositions or formulations containing caprylyl glycol, iodopropynyl butylcarbamate and phenoxyethanol the by weight ratios are preferably in the range of 55/1/100 to 200/1/400.

The amount of caprylyl glycol or its analogs in the formulations or compositions according to this invention may vary, but will be selected such that the combination thereof with the preservatives has an effective preservative activity. It has been found that good results are obtained when using concentrations in the range from about 0.1 to 30% weight/weight (w/w) of the composition. In particular, the concentration of caprylyl glycol or its analog should be in the range from 0.1 to 20 %, more in particular from 0.2 to 10 %, preferably from 0.5 to 5 % or 0.5 to 2 %, all percentages being w/w. A particularly preferred concentration is 1 % w/w.

The amount of the preservative agents, and in particular of IPBC, in the compositions or formulations according to this invention may vary but it has been found that good results are obtained when using concentrations in the range from about 0.001 to 1% w/w of the composition, in particular from 0.001 to 0.5 %, or from 0.001 to 0.25 %, in particular from 0.001 to 0.100 %, or from 0.001 to 0.050 %, all percentages being w/w. A particular preferred concentration is in the range of 0.010 to 0.025 % w/w. Or expressed differently, the concentration of the preservative agent and in particular of IPBC in the formulation is in the range 1 to 250 ppm, or 1 to 200 ppm, more preferably in the range of 10 to 200 ppm, 'ppm' also referring to w/w.

Of special interest is a composition or formulation containing about 1 % w/w of caprylyl glycol and about 0.018 % w/w of IPBC, having a broad anti-microbial spectrum that clearly shows better results than with IPBC alone.

In the compositions or formulations containing caprylyl glycol, iodopropynyl butylcarbamate and phenoxyethanol the concentration of caprylyl glycol preferably is in the range of 0.1 % to 30 %, more preferably in the range of 0.1 % to 5 %, or of 0.1 % to 2 %, or of 0.1 % to 1 %, a particularly preferred concentration being 0.5 %. The concentration of iodopropynyl butylcarbamate preferably is in the range of 0.001 % to 1 %, more preferably 0.001 % to 0.1 % or 0.001 to 0.01 %. The concentration of phenoxyethanol preferably is in the range of 0.1 % to 3 %, more preferably in the range of 0.1 % to 2 % or of 0.5 % to 1 %. Percentages in this and the following paragraph are weight by weight.

Preferably in these compositions or formulations the by weight ratio of IPBC to phenoxyethanol is about 1/100 and the concentration of both said agents taken together is in the range of 0.01 % to 3 %, more preferably in the range of 0.1 % to 2 % or of 0.5 % to 1 %. A particularly preferred concentration of both said agents is 0.8 %.

This invention further relates to topical formulations containing a composition as defined herein. Topical compositions comprise as well dermatological formulations (or topical pharmaceutical formulations), as cosmetical formulations. Said topical formulations may further contain other ingredients or additives used in dermatological or in cosmetical formulations, including other active ingredients.

The formulations of this invention are formulated into forms that are useful in personal care products that are available in many different forms. They include creams, lotions, pastes, liquids, aerosols, shampoos, gels, wipes, bars, sticks, powders and granules any or all of which can be referred to as 'cosmetic products' or 'cosmetics' and are intended for topical application to the skin including the scalp and the mucosa including the lips.

The compositions or formulations according to the present invention preferably are formulated into forms that are useful in sunscreen products. In particular they are formulated as in emulsions.

The compositions and in particular the formulations according to the present invention may additionally contain further ingredients or additives such as surfactants, emulsifiers, consistency factors, conditioners, emollients, skin caring ingredients, moisturizers, thickeners, glidants, lubricants, fillers, binding agents, anti-oxidants, preservatives, active ingredient, in particular dermatologically active ingredients, fragrances and the like. Active ingredients as mentioned herein comprise, for example, anti-inflammatories, antiallergics and the like agents. Active ingredients suited for topical applications are particularly preferred.

Suitable surfactants comprise:
alkyl sulfates e.g. sodium lauryl sulfate, ammonium lauryl sulfate, sodium cetearyl sulfate,
alkyl sulfoacetates e.g. sodium lauryl sulfoacetate,
alkyl ether sulfates e.g. sodium laureth sulfate, sodium trideceth sulfate, sodium oleth sulfate, ammonium laureth sulfate
alkyl ether sulfosuccinates e.g. disodium laureth sulfosuccinate
alkyl glycosides e.g. decyl glucoside, lauryl glucoside,
alkyl isethionates
amphoterics e.g. cocamidopropyl betaine, sodium cocoamphoacetate, sodium lauroamphoacetate, disodium lauroamphodiacetate, disodium cocoamphodiacetate, sodium lauroamphopropionate, disodium lauroamphodipropionate, potassium or ammonium slats of the aforementioned amphoterics, capryl/capramidopropyl betaine, undecylenamidopropyl betaine, lauramidopropyl betaine and
fatty alcohol polyglycol ethers.

Suitable emulsifiers are e.g. anionics as salts of fatty acids e.g. sodium stearate or sodium palmitate, organic soaps e.g. mono-, di- or triethanolaminoleate, sulfated or sulfonated compounds e.g. sodium lauryl sulfate or sodium cetyl sulfonate, saponines, lamepones; cationics as quaternary ammonium salts; nonionics as fatty alcohols, fatty acid ester with saturated or unsaturated fatty acids, polyoxyethylenesters or polyoxyethylenethers of fatty acids, polymers from ethylene oxide and propylene oxide or propylene glycol, amphotherics as phosphatides, proteines as gelatine, casein alkylamidobetaines, alkyl betaines and amphoglycinates, alkyl phosphates, alkylpolyoxyethylene phoaphates or the corresponding acids, silicone derivatives, e.g. alkyl dimethiconecoplyol.

Suitable consistency factors are e.g. fatty alcohols or their mixtures with fatty acid esters, e.g. acetylated lanolin alcohol, aluminum stearates, carbomer, cetyl alcohol, glyceryl oleate, glyceryl stearate, glyceryl stearate (and) PEG 100 stearate, magnesium stearate, magnesium sulfate, oleic acid, stearic acid, stearyl alcohol, myristyl myristate, isopropyl palmitate, beeswax and synthetic equivalents thereof, carbomers, etc. Suitable conditioners are e.g. alkylamido ammonium lactate, cetrimonium chloride and distearoylethyl hydroxyethylmonium methosulfate and cetearyl alcohol, cetyl dimethicone, cetyl ricinoleate, dimethicone, laureth-23, laureth-4, polydecene, retinyl palmitate, quaternised protein hydrolysates, quaternised cellulose and starch derivatives, quaternised copolymers of acrylic or methacrylic acid or salts, quaternised silicone derivatives.

Suitable emollients are e.g. cetearyl isononanoate, cetearyl octanoate, decyl oleate, isooctyl stearate, coco caprylate/caprate, ethylhexyl hydroxystearate, ethylhexyl isononanoate, isopropyl isostearate, isopropyl myristate, oleyl oleate, hexyl laurate, paraffinum liquidum, PEG-75 lanolin, PEG-7 glyceryl cocoate, petrolatum, ozokerite, cyclomethicone, dimethicone, dimethicone copolyol, dicaprylyl ether, butyrospermum parkii, buxus chinensis, canola, carnauba cera, copernicia cerifera, oenothera biennis, elaeis guineensis, prunus dulcis, squalane, zea mays, glycine soja, helianthus annuus, lanolin, hydrogenated castor oil, hydrogenated coconut oil, hydrogenated polyisobutene, sucrose cocoate, stearoxy dimethicone, lanolin alcohol, isohexadecane.

Suitable skin caring ingredients are e.g. plant extracts, bisabolol, antiinflammatory agents, urea, allantoin, panthenol and panthenol derivatives, phytantriol, vitamines A, B5, E, C, D, ceramides of animal or plant origin, lecithins, etc.

Suitable moisturizers are e.g. butylene glycol, cetyl alcohol, dimethicone, dimyristyl tartrate, glucose, glycereth-26, glycerin, glyceryl stearate, hydrolyzed milk protein, lactic acid, lactose and other sugars, laureth-8, lecithin, octoxyglycerin, PEG-12, PEG-135, PEG-150, PEG-20, PEG-8, pentylene glycol, hexylene glycol, phytantriol, polyquaternium-39, PPG-20 methyl glucose ether, propylene glycol, sodium hyaluronate, sodium lactate, sodium PCA, sorbitol, succinoglycan, synthetic beeswax, tri-C₁₄₋₁₅ alkyl citrate, starch.

Suitable thickeners are e.g. acrylates/steareth-20 methacrylate copolymer, carbomer, carboxymethyl starch, cera alba, dimethicone/vinyl dimethicone crosspolymer, propylene glycol alginate, hydroxyethylcellulose, hydroxypropyl methylcellulose, silica, silica dimethyl silylate, xanthan gum, hydrogenated butylene/ethylene/styrene copolymer.

Suitable lubricants are e.g. adipic acid, fumaric acid and its salts, benzoic acid and its salts, glycerine triacetate, sodium or magnesium lauryl sulfate, magnesium stearate, solid polyethylenglycol, polyvinylpyrrolidone, boric acid, monolaurate or - palmitate, myristyl alcohol, cetyl alcohol, cetylstearyl alcohol, talcum, calcium or magnesium salts of higher fatty acids, mono-, di- or triglycerides of higher fatty acids, polytetrafluorethylen.

Suitable anti-oxidants are e.g. sulfites, e.g. sodium sulfite, tocopherol or derivates thereof, ascorbic acid or derivates thereof, citric acid, propyl gallate, chitosan glycolate, cysteine, N-acetyl cysteine plus zinc sulfate, thiosulfates, e.g. sodium thiosulfate, polyphenoles and the like.

The compositions may further contain active ingredients, e.g. anti-microbials such as complexes of PVP and hydrogen peroxide, anti-inflammatories as, plant extracts, bisabolol, panthenol, tocopherol, actives for anti-stinging, anti-irritants, antidandruffs, for anti-ageing e.g. retinol, melibiose etc. Other suitable actives are e.g. Medicago officinalis, Actinidia chinensis, allantoin, Aloe barbadensis, Anona cherimolia, Anthemis nobilis, Arachis hypogaea, Arnica montana, Avena sativa, beta-carotene, bisabolol, Borago officinalis, butylene glycol, Calendula officinalis, Camellia sinensis, camphor, Candida bombicola, capryloyl glycine, Carica papaya, Centaurea cyanus, cetylpyridinium chloride, Chamomilla recutita, Chenopodium quinoa, Chinchona succirubra, Chondrus crispus, Citrus aurantium dulcis, Citrus grandis, Citrus limonum, Cocos nucifera, Coffea arabica, Crataegus monogina, Cucumis melo, dichlorophenyl imidazoldioxolan, Enteromorpha compressa, Equisetum arvense, ethoxydiglycol, ethyl panthenol, farnesol, ferulic acid, Fragaria chiloensis, Gentiana lutea, Ginkgo biloba, glycerin, glyceryl laurate, Glycyrrhiza glabra, Hamamelis virginiana, heliotropine, hydrogenated palm glycerides, citrates, hydrolyzed castor oil, hydrolyzed wheat protein, Hypericum perforatum, Iris florentina, Juniperus communis, lactis proteinum, lactose, Lawsonia inermis, linalool, Linum usitatissimum, lysine, Magnesium aspartate, magnifera indica, Malva sylvestris, mannitol, mel, Melaleuca alternifolia, Mentha piperita, menthol, menthyl lactate, Mimosa tenuiflora, Nymphaea alba, olaflur, Oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, Persea gratissima, petrolatum, potassium aspartate, potassium sorbate, propylene glycol, Prunus amygdalus dulcis, prunus armeniaca, Prunus persica, retinyl palmitate, Ricinus communis, Rosa canina, Rosmarinus officinalis, rubus idaeus, salicylic acid, Sambucus nigra, sarcosine, Serenoa serrulata, Simmondsia chinensis, sodium carboxymethyl betaglucan, sodium cocoyl amino acids, sodium hyaluronate, sodium palmitoyl proline, stearoxytrimethylsilane, stearyl alcohol, sulfurized TEA-ricinoleate, talcum, thymus vulgaris, Tilia cordata, tocopherol, tocopheryl acetate, trideceth-9, Triticum vulgare, tyrosine, undecylenoyl glycine, urea, Vaccinium myrtillus, valine, zinc oxide, zinc sulfate and the like.

The combination of caprylyl glycol or an analog thereof and the two said preservatives can be used in emulsions, both oil in water, and water in oil, in aqueous solutions, in PIT (phase inversion temperature) emulsions, in oily solutions, in foaming cosmetic formulations (foams), and in multiple emulsions (for instance triple emulsions such as water-in-oil-in-water emulsions).

The compositions or formulations of the invention can be formulated as creams, gels, liquids or lotions suited for suncare products. The compositions comprising caprylyl glycol, IPBC and phenoxyethanol are particularly useful in products containing inorganic powders for application in suncare products.

The compositions or formulations of the present invention are generally prepared by mixing the caprylyl glycol or the analog thereof, and the two preservatives, and optional other ingredients. An example of such other ingredients are perfumes. The ingredients may also be mixed groupwise and subsequently the thus obtained mixtures may be mixed together in a final step.

In a further aspect, this invention is concerned with synergistic effects between three agents in particular caprylyl glycol or an analog on the one hand, and a combination of IPBC and phenoxyethanol on the other. Said synergistic effects occur in terms of anti-microbiological activity, as well as anti-microbiological spectrum, showing a better efficacy than the two components alone.

Hence in still a further aspect the present invention provides synergistic chemical compositions comprising caprylyl glycol, IPBC and phenoxyethanol. In these synergistic compositions the concentrations of caprylyl glycol or its analogs, and of IPBC, and of phenoxyethanol, as well as the by weight ratios of said glycol or IPBC or phenoxyethanol can be as mentioned herein.

The synergistic effect in the compositions of the invention can be demonstrated by measuring the antimicrobial activity and in particular the activity against Staphylococcus aureus of octanediol alone and measuring the same of a mixture of IPBC and phenoxyethanol. Subsequently mixtures of all three components in various concentrations, in particular in the concentrations and the w/w ratios mentioned herein above, are tested for their activity against the same micro-organism.

The use of 1,2-octanediol with IPBC and phenoxyethanol in suncare products and other topical formulations results in both a broad anti-microbial protection and a good skin tolerance. The anti-microbial protection is as well against bacteria as fungi, in particular against species such as, for example, Pseudomonas aeruginosa, Escherichia coli, Staphyococcus aureus, Candida albicans, Aspergillus niger and the like and especially against Staphylococcus aureus.

The chemical compositions of the invention are particularly attractive for application in suncare formulations or products, with the additional advantage that they can be used on sensitive skins.

These compositions are particularly useful in sunscreen formulations or products that contain inorganic components, more specifically powdery inorganic components, also referred to as mineral sunscreens, such as for example titanium dioxide powders. They can be applied in sunscreen formulations that contain such powdery components either alone or in combination with sunscreen filters of organic nature. More particularly they can be applied in formulations having a high SPF, i.e. an SPF of 15 or 25 or higher.

The present formulations are further attractive since the agents therein have a particularly good anti-microbial action against Staphylococcus aureus. Thus in a further aspect, this invention concerns the use of the formulations as defined herein for combating microorganisms or preventing the growth thereof on human skin. In that sense the combination of the agents mentioned herein could have dermatological or pharmaceutical applications and to that purpose could be formulated in appropriate formulations for topical dermatological or pharmaceutical applications.

In still a further aspect of the invention the chemical compositions as defined herein can also be used in cosmetic formulations and products that contain inorganic powders, in particular large amounts of inorganic powders. Thus the invention concerns cosmetic formulations containing a composition as defined herein and an amount of inorganic powders, said amount being at least 20%, in particular at least 30%, preferably at least 50%, or at least 70%, or at least 80%.

Examples of the latter are skin compatible transition element oxides, e.g. (red, yellow and black) iron oxides, zinc oxides, titanium oxides, and the like, and mixtures thereof. These and other powders can be used as pigments and are described in the Handbook of Pharmaceutical Excipients, The Pharmaceutical Press, Second Edition, pp. 131 and 529. These cosmetic formulations take a variety of forms, in particular the types described above, they in particular can be oily, aqueous or they can be emulsions, either oil in water, the latter also including PIT formulations, water in oil, or so-called mixed emulsions such as water/oil/water.

Examples of such formulations or products are make-up products, e.g. foundations. The ratios of the amounts of the preservative components as well as the concentration thereof in the cosmetic formulations in which they are used, will be as defined hereinabove for the suncare formulations.

The invention is further illustrated by the following examples.

### Example 1:

| Phase I | | | |
|---|---|---|---|
| Phases | Ingredients | % | Weight (g) |
| A | PEG-100 stearate and glyceryl stearate | 3 | 75 |
| | potassium cetyl phosphate | 1.3 | 32.5 |
| | cetearyl alcohol | 1 | 25 |
| | glycerin monomyristate | 2 | 50 |
| | | | |
| B | PVP hexadecene copolymer | 3 | 75 |
| | | | |
| C | Tocopherol Acetate/Vit. E Acetate | 0.5 | 12.5 |
| | | | |
| D | C₁₂₋₁₅ alkyl benzoate | 7 | 175 |
| | isononyl isononanoate | 3 | 75 |
| | | | |
| E | ethylhexyl methoxycinnamate | 8 | 200 |
| | butylmethoxy dibenzoylmethane | 3 | 75 |
| | 4-methyl benzylidene camphor | 4 | 100 |
| | | | |
| F | TiO₂ coated with Al-stearate/stearic acid | 13 | 75 |
| | | | |

| Phase II | | | |
|---|---|---|---|
| Phases | Ingredients | % | Weight (g) |
| G | demineralized water | 48,96 | 1224 |
| | acrylate/C₁₀₋₂₀ alkylacrylate crosspolymer | 0.1 | 2.5 |
| | | | |
| H | demineralized water | 1 | 25 |
| | sodium hydroxide | 0.02 | 0.5 |
| | | | |
| I | Hydroxyethyl cellulose | 0.5 | 12.50 |
| | disodium EDTA | 0.1 | 2.5 |
| | IPBC | 0.1 | 2.5 |
| | caprylyl glycol (octanediol) | 0.5 | 12.5 |
| | | | |
| J | phenoxyethanol | 1 | 25 |
| | butylene glycol | 3 | 75 |
| | glycerine | 2 | 50 |
| | | | |
| K | cyclomethicone | 2.5 | 62.5 |
| | | | |
| L | Ginkgo Biloba | 0.05 | 1.25 |
| | carboxymethyl betaglucan | 0.25 | 6.25 |
| | | | |
| M | demineralized water | 1 | 25 |
| | citric acid | 0.12 | 3 |
| | Total | 100 | 2500 |

### Procedure

In a first step, the oily phase is prepared by mixing A+ B+ C+ D+ E+ F at 80°C for at least 30 minutes, then at 90°C for 10 minutes.

The acrylate copolymer is dispersed in water under intense stirring while raising the temperature from room temperature to 40°C. The mixture is neutralized by adding sodim hydroxide and water. Subsequently the ingredients I and J are added while stirring.

The oily phase is heated to about 92% C and is added to the aqueous phase at 75°C while stirring. The whole is cooled to 70°C whereupon cyclomethicone is added. After further cooling to 35°C the ingredients L and M are added while stirring.

### Example 2:

| Ingredients | % | Weight (g) |
|---|---|---|
| Phase A | | |
| PEG-100 stearate & glyceryl stearate | 3 | 15 |
| Potassium cetyl phosphate | 1.3 | 6.5 |
| Cetearyl alcohol | 0.3 | 1.5 |
| Glycerin monomyristate | 0.5 | 2.5 |
| Butyrospermium parkii (shea butter) | 0.5 | 2.5 |
| PVP-Eicosene copolymer | 3 | 15 |
| Tocopheryl acetate | 0.5 | 2.5 |
| Disodium EDTA | 0.1 | 0.5 |
| | | |
| C ₁₂₋₁₅ alkyl benzoate | 5 | 25 |
| Hydrogenated polyisobutene | 3 | 15 |
| Dimethicone | 2 | 10 |
| Ethylhexylmethoxycinnamate camphor | 8.5 | 42.5 |
| 4-methylbenzilidene camphor | 4 | 20 |
| Bis octoxyphenol methoxyphenyl triazine | 2 | 10 |
| TiO ₂ coated with Al-stearate/stearic acid | 3 | 15 |
| | | |

| Phase B | | |
|---|---|---|
| | | |
| Demineralized water | 42 | 210 |
| Xanthan gum | 0.2 | 1 |
| Dimethicone copolyol phosphate | 0.8 | 4 |
| Iodopropynyl butyl carbamate & phenoxyethanol | 0.8 | 4 |
| Octanediol | 0.5 | 2.5 |
| Butylene glycol | 5 | 25 |
| Glycerine | 2 | 10 |
| | | |

| Phase C | | |
|---|---|---|
| | | |
| Cyclomethicone | 3 | 15 |
| Ammonium polyacrylate/isohexadecane/PEG-40 castor oil | 1 | 5 |
| Methylene bisbenzotriazolyl tetramethylbutylphenol | 6 | 30 |
| Dimethicone copolyol polyacrylate | 1 | 5 |
| Al starch/octenyl succinate | 1 | 5 |

### Procedure:

The oily phase A is prepared by mixing the ingredients listed above at a temperature of 85° - 92°. The other ingredients are added subsequently. The oily phase A is heated to 85° C and is added to phase B at 80° C while stirring. The whole is allowed to cool to 70° C whereupon phase C is added. The whole is stirred and allowed to cool to room temperature.

## Claims

1. A chemical composition containing at least one C₅₋₂₀ alkanediol wherein the hydroxy groups are vicinally substituted and wherein one hydroxy group is substituted at an end carbon and the other on the carbon next thereto and/or at least one 1,2-octanediol substituted with 1, 2 or 3 C₁₋₄ alkyl groups or mixtures thereof, and a combination of phenoxyethanol and iodopropynyl butylcarbamate.

2. The chemical composition according to claim 1 wherein the C₅₋₂₀ alkanediol is caprylyl glycol.

3. The composition according to claim 1 or 2 wherein the weight ratio iodopropynyl butylcarbamate and phenoxyethanol is in the range of about 1:90 to about 1:400.

4. The composition according to claim 3, wherein the said weight ratio is within the range of about 1:90 to about 1:200.

5. The composition according to claim 4, wherein the said weight ratio is about 1:100.

6. The composition according to one of the preceding claims further containing an amount of inorganic powders.

7. The chemical composition according to claim 6 wherein the inorganic powder is titanium dioxide.

8. A topical formulation containing a chemical composition as claimed in any of claims 1 to 7.

9. The topical formulation according to claim 8 which is a sunscreen formulation.

10. The topical formulation according to claim 8 or 9 which is a foundation.

11. A sun care product comprising a formulation as claimed in any of claims 8 to 10.

12. The use of a chemical composition according to one of claims 1 to 7 as a preservative agent, in particular in a topical formulation.

## Patentansprüche

1. Chemische Zusammensetzung, enthaltend zumindest ein C₅₋₂₀-Alkandiol, worin die Hydroxygruppen vicinal substituiert sind und worin die eine Hydroxygruppe an einem endständigen Kohlenstoff und die andere an dem diesem zunächst gelegenen Kohlenstoff substituiert ist, und/oder zumindest ein 1,2-Octandiol, das mit einer, zwei oder drei C₁₋₄-Alkylgruppen substituiert ist oder Mischungen hiervon, sowie eine Kombination von Phenoxyethanol und Iodpropinylbutylcarbamat.

2. Chemische Zusammensetzung nach Anspruch 1, **dadurch gekennzeichnet, dass** das C₅₋₂₀-Alkandiol Caprylylglykol ist.

3. Zusammensetzung nach Anspruch 1 oder 2, **dadurch gekennzeichnet, dass** das Gewichtsverhältnis von Iodpropinylbutylcarbamat und Phenoxyethanol im Bereich von etwa 1 : 90 bis etwa 1 : 400 liegt.

4. Zusammensetzung nach Anspruch 3, **dadurch gekennzeichnet, dass** das besagte Gewichtsverhältnis innerhalb des Bereiches von etwa 1 : 90 bis etwa 1 : 200 liegt.

5. Zusammensetzung nach Anspruch 4, **dadurch gekennzeichnet, dass** das besagte Gewichtsverhältnis bei etwa 1 : 100 liegt.

6. Zusammensetzung nach einem der vorangehenden Ansprüche, ferner enthaltend eine Menge an anorganischem Pulver.

7. Chemische Zusammensetzung nach Anspruch 6, **dadurch gekennzeichnet, dass** das anorganische Pulver Titandioxid ist.

8. Topische Formulierung, enthaltend eine chemische Zusammensetzung wie in einem der Ansprüche 1 bis 7 beansprucht.

9. Topische Formulierung nach Anspruch 8, die eine Sonnenschutzformulierung darstellt.

10. Topische Formulierung nach Anspruch 8 oder 9, die eine Grundierung darstellt.

11. Sonnenpflegeprodukt, enthaltend eine Formulierung wie in einem der Ansprüche 8 bis 10 beansprucht.

12. Verwendung einer chemischen Zusammensetzung nach einem der Ansprüche 1 bis 7 als Konservierungsmittel, insbesondere in einer topischen Formulierung.

## Revendications

1. Composition chimique contenant au moins un alcanediol en C₅₋₂₀ dans lequel les groupes hydroxyle sont substitués de façon contiguë et dans lequel un groupe hydroxyle est substitué sur un carbone d'extrémité et l'autre sur le carbone à côté de celui-ci et/ou au moins un 1,2-octanediol substitué par 1, 2 ou 3 groupes alkyle en C₁₋₄ ou mélanges de ceux-ci, et une combinaison de phénoxyéthanol et de butylcarbamate d'iodopropynyle.

2. Composition chimique selon la revendication 1 dans laquelle l'alcanediol en C₅₋₂₀ est le glycol caprylique.

3. Composition selon la revendication 1 ou 2 dans laquelle le rapport pondéral du butylcarbamate d'iodopropynyle au phénoxyéthanol se situe dans la fourchette d'environ 1:90 à environ 1:400.

4. Composition selon la revendication 3 dans laquelle ledit rapport pondéral se situe dans la fourchette d'environ 1:90 à environ 1:200.

5. Composition selon la revendication 4 dans laquelle ledit rapport pondéral est d'environ 1:100.

6. Composition selon l'une quelconque des revendications précédentes contenant en outre une certaine quantité de poudres inorganiques.

7. Composition chimique selon la revendication 6 dans laquelle la poudre inorganique est du dioxyde de titane.

8. Formulation topique contenant une composition chimique selon l'une quelconque des revendications 1 à 7.

9. Formulation topique selon la revendication 8 qui est une formulation d'écran solaire.

10. Formulation topique selon la revendication 8 ou 9 qui est un fond de teint.

11. Produit solaire comprenant une formulation selon l'une quelconque des revendications 8 à 10.

12. Utilisation d'une composition chimique selon l'une quelconque des revendications 1 à 7 comme conservateur, en particulier dans une formulation topique.
